# NOUVEAU FASCICULE DE BREVET EUROPEEN

(11) **EP 0 772 469 B2**
(45) Date de publication et mention de la décision concernant l'opposition: **22.02.2006**
(45) Mention de la délivrance du brevet: 11.09.2002
(21) Numéro de dépôt: 96914357.7
(22) Date de dépôt: 30.05.1996
(51) Int. Cl.: A61M 1/16, A61M 1/36

(54) **PROCEDE ET DISPOSITIF POUR LE RIN AGE D'UN APPAREIL A MEMBRANE**
VERFAHREN UND VORRICHTUNG ZUR SPÜLUNG EINES MEMBRANAPPARATS
METHOD AND DEVICE FOR FLUSHING A MEMBRANE APPARATUS

(30) Priorité: 30.05.1995 IT TO950442
(43) Date de publication de la demande: 14.05.1997
(73) Titulaire: GAMBRO HOSPAL (Schweiz) AG, 4001 Basel (CH)
(72) Inventeur: BARDELLI, Francesco, I-41036 Medolla (IT); PUPPINI, Anna, I-41033 Concordia S/S (IT)
(74) Mandataire: Sutto, Luca
(86) Numéro de dépôt international: PCT/IB1996/000530
(87) Numéro de publication internationale: WO 1996/038189

(56) Documents cités:
- EP-A- 0 543 172
- EP-A- 0 560 368
- DE-C- 2 838 414
- US-A- 4 900 152
- US-A- 5 004 548
- US-A- 5 041 215
- "Blutreinigungsverfahren", publié par H.E.Franz, 2ième edition, Georg-Thieme-Verlag, Stuttgart, 1981, pages 50-62

## Description

La présente invention concerne un procédé et un dispositif pour le rinçage initial d'un appareil de traitement du sang.

Il est connu qu'avant de commencer un traitement de dialyse il faut rincer et un remplir le dialyseur (étape dite de "priming" ou amorçage) en faisant passer une solution de rinçage dans les compartiments du dialyseur. Pour effectuer un tel rinçage, il a été proposé diverses méthodes. Par exemple, dans le brevet US-A-5 004 548 il est décrit une méthode consistant à raccorder la sortie du compartiment pour le sang du dialyseur à l'entrée du compartiment pour le liquide de dialyse et à faire circuler en série, à travers le compartiment sang et le compartiment liquide de dialyse une solution physiologique stérile provenant d'une poche. La sortie du compartiment liquide de dialyseur est reliée à une poche de collecte et d'évacuation du liquide de rinçage.

Selon un procédé de rinçage connu, le liquide de rinçage n'est pas recueilli dans une poche de collecte mais il est éliminé par les canalisations d'évacuation de liquide usé de l'appareil de dialyse. Dans ce but, le raccord de sortie du compartiment liquide de dialyse est relié, par l'intermédiaire d'une conduite et d'une vanne électromagnétique, à une canalisation d'évacuation prévue dans l'appareil de dialyse, à proximité de la sortie d'un l'ultrafiltre. En même temps, un liquide de dialyse est mis en circulation dans l'uttrafiltre, de sorte que le liquide de rinçage du dialyseur et le liquide de dialyse en préparation sont ainsi évacués ensemble dans la canalisation d'évacuation.

Cette solution a l'avantage de permettre de réaliser une économie notable, due à la suppression de la poche dé collecte et aux frais de mise au rebut de celle-ci et d'améliorer le rinçage du dialyseur. Il n'existe en outre aucun risque de contamination du dialyseur ou des parties stériles de l'appareil par le liquide non stérile, car il est prévu une séparation complète entre les parties stérile et non stérile de l'appareil..

Pour assurer un rinçage adéquat du dialyseur, il faut éliminer des bulles d'air éventuelles présentes sur la membrane du dialyseur. Pour atteindre ce but, actuellement, la personne chargée de mettre en oeuvre la dialyse frappe de légers coups sur le dialyseur pour que les bulles se décollent de la membrane et soient évacuées avec le liquide de rinçage.
Le document US5004548 montre un système de rinçage d'un appareil de traitement du sang ayant un premier et un second compartiments séparés par une membrane semi-perméable, consistant à: agencer un circuit de rinçage en reliant une entrée du premier compartiment à une source de liquide de rinçage, en reliant la sortie du premier compartiment à une entrée du second compartiment et en reliant la sortie du second compartiment à des moyens d'évacuation; faire circuler du liquide de rinçage dans le circuit de rinçage par des moyens de pompage disposés sur le circuit de rinçage du côté de l'entrée du compartiment.
Le document « Blutreinigungsverfahren - Georg Thieme Verlag Stuttgart . New York - 1981 - 5. Technik der Hämodialyse » montre un système de rinçage d'un appareil de traitement du sang ayant un premier et un second compartiments séparés par une membrane semi-perméable, le système consistant à faire circuler du liquide de rinçage dans le circuit de rinçage, créer des ondes de pression à intervalles de temps dans le circuit de rinçage de façon à décoller des bulles d'air adhérant à une paroi interne de l'appareil de traitement de sang et à évacuer ces bulles d'air dans le liquide de rinçage, les ondes de pression étant créées en ouvrant et en fermant alternativement des moyens d'obturation disposés sur le circuit de rinçage.

Un but de l'invention consiste à perfectionner le procédé de rinçage indiqué plus haut, de manière à le rendre complètement automatique, tout en conservant les avantages du procédé de rinçage connu, décrit plus haut.

Pour atteindre ce but, on prévoit, conformément à l'invention, un procédé de rinçage d'un appareil de traitement du sang ayant un premier et un second compartiments séparés par une membrane semi-permeable, selon la revendication 2.

Conformément à l'invention, on prévoit aussi un dispositif de rinçage d'un appareil de traitement du sang ayant un premier et un second compartiments séparés par une membrane semi-perméable, selon la revendication 1.

D'autres avantages et caractéristiques de l'invention apparaîtront à la lecture de la description d'un mode de réalisation préféré de l'invention, faite à titre non limitatif et en regard des dessins annexés, où :
- la figure 1 représente schématiquement le circuit hydraulique d'une machine de dialyse,
- la figure 2 illustre l'allure des courbes de certaines grandeurs mesurées dans le circuit de la figure 1.

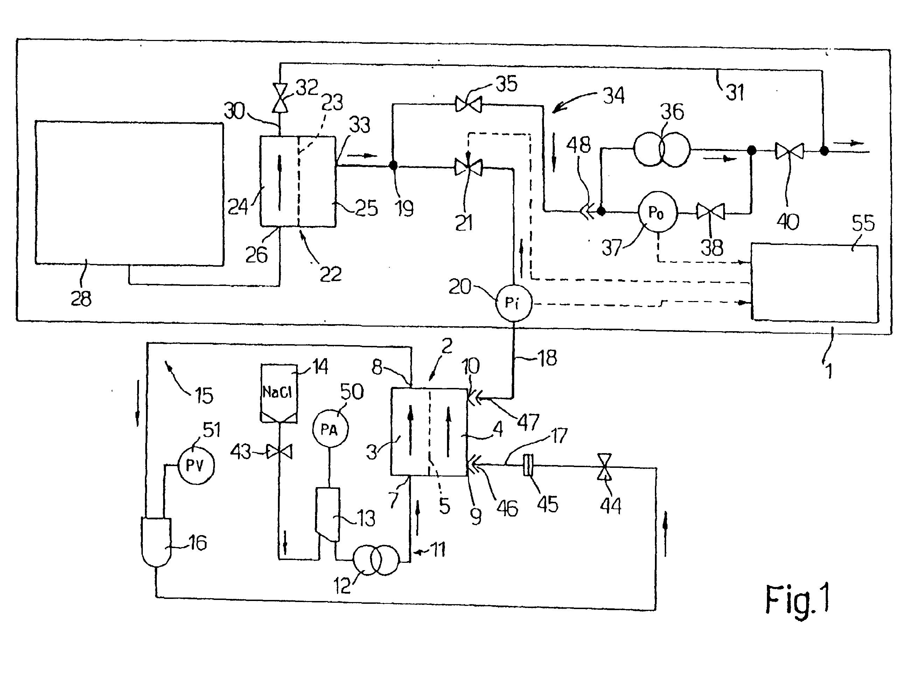

Sur la figure 1, il est représenté de façon schématique le circuit hydraulique d'une machine de dialyse 1 relié à un dialyseur 2 pour l'étape de rinçage. Il a été omis sur la figure les raccordements et les éléments qui n'interviennent pas dans le procédé de rinçage selon l'invention.

Le dialyseur 2 comprend un compartiment sang 3 et un compartiment liquide de dialyse 4, séparés par une membrane semi-perméable 5. Le compartiment sang 3 comporte une entrée 7 et une sortie 8 ; le compartiment liquide de dialyse 4 comporte une entrée 9 et une sortie 10. L'entrée 7 du compartiment sang 3 est reliée à une poche 14 de liquide de rinçage (solution physiologique stérile) au moyen d'une ligne artérielle 11 munie d'une pompe péristaltique 12 (pompe à sang) et une chambre d'expansion 13 ; la sortie 8 du compartiment sang est reliée à l'entrée 9 du compartiment liquide de dialyse au moyen d'une ligne veineuse 15, munie d'un piège à bulles 16, et d'une portion de canalisation 17. La sortie 10 du compartiment liquide de dialyse est reliée à la sortie d'un ultrafiltre 22 (point de jonction 19) au moyen d'une canalisation de raccordement 18 munie d'un capteur 20 de pression Pi et d'une vanne électromagnétique 21.

L'ultrafiltre 22 est divisé par une membrane semi-perméable 23 en deux chambres : une chambre d'entrée 24 et une chambre de sortie 25. La chambre d'entrée 24 comporte une entrée 26 reliée à une unité de préparation de liquide de dialyse 28 et une sortie 30 reliée à une canalisation de dérivation 31 munie d'une vanne électromagnétique 32. La chambre de sortie 25 comporte une sortie 33 reliée à la canalisation de raccordement 18 et à une canalisation d'évacuation 34.

La canalisation d'évacuation 34 comporte un premier tronçon et un second tronçon de canalisation reliés par un raccord 48. Le premier tronçon, qui est relié directement à la sortie 33 de l'ultrafiltre 22, est muni d'une première vanne électromagnétique 35. Le second tronçon est muni d'une pompe à engrenage 36 et d'une deuxième vanne électromagnétique 40. Une portion de canalisation munie d'un capteur de pression P0 et d'une troisième vanne électromagnétique 38 est montée en dérivation à la pompe 36.

La canalisation de dérivation 31. dont une extrémité est connectée à la sortie 30 de la première chambre de l'ultrafiltre 22, est reliée, à son autre extrémité. à la canalisation d'évacuation 31, en aval de la deuxième vanne électromagnétique 40.

Il est également représenté schématiquement sur la figure 1 différentes pinces de fermeture (clamps) et différents raccords disposés sur les tuyauteries. En particulier, sur la conduite artérielle 11, entre la pompe sang 12 et la poche 14, il est prévu une pince de fermeture 43 et une pince analogue 44 est disposée sur la conduite veineuse 15 en aval du piège à bulles 16. Un raccord 45 est prévu pour connecter la canalisation veineuse 15 à la portion de canalisation 17; un raccord 46 est prévu pour connecter la portion de canalisation 17 à l'entrée 9 de la chambre liquide de dialyse du dialyseur 2 ; un raccord 47 est prévu pour connecter la sortie 10 de la chambre liquide de dialyse 4 à la canalisation de raccordement 18 ; et, le raccord 48 disposé sur la canalisation d'évacuation 34 est prévu pour connecter le deuxième tronçon de la canalisation d'évacuation 34 à l'entrée 9 de la chambre de liquide de dialyse, à la fin de l'étape de lavage, pour procéder à la dialyse.

Un capteur 50 de pression PA est relié à la chambre d'expansion 13, et un capteur 51 de pression veineuse PV est relié au piège à bulles 16.

La vanne 21 de la canalisation de raccordement 18 a pour fonction d'assurer la protection totale du dialyseur 2 contre toute contamination éventuelle de la part du liquide de dialyse au cours de l'étape de rinçage. La vanne 21 est commandée de façon que la pression dans la canalisation de raccordement 18 reste toujours supérieure à la pression dans la canalisation d'évacuation 34 et qu'un reflux du liquide de dialyse en cours de préparation vers le dialyseur 2 ne puisse pas se produire. A cet effet, les pressions Pi et P0, mesurées au moyen des capteurs de pression 20, 37 dans la canalisation de raccordement 18 et dans la canalisation d'évacuation 34 sont contrôlées de façon continue par une unité de commande et de contrôle 55 et la valve 21 est ouverte et fermée en fonction de leurs valeurs respectives. La première vanne électromagnétique, 35 est constamment ouverte pour permettre l'évacuation du liquide de dialyse en préparation au cours de l'étape de rinçage et elle estfermée lorsque la dialyse est en cours. Les vannes 32, 38 et 40 servent à fermer ou à ouvrir les canalisations correspondantes en fonction de conditions de sécurité prédéterminées. L'unité de commande 55 qui reçoit les pressions Pi et P0 mesurées par les capteurs 20 et 37, commande toutes les vannes du circuit (en particulier la vanne 21).

Le procédé de rinçage se déroule de la façon suivante. Initialement, la vanne électromagnétique 21 est fermée et la première vanne 35 de la canalisation d'évacuation 34 est ouverte. Après le raccordement du circuit hydraulique de la façon décrite et représentée, la pompe artérielle 12 est mise en fonctionnement et elle aspire le liquide de rinçage (solution physiologique) de la poche 14 et le refoule dans la conduite artérielle 11 et dans le compartiment sang 3 du dialyseur 2, comme représenté par les flèches sur la figure 1. Le liquide s'écoule ensuite dans la conduite veineuse 15 et la portion de canalisation 17 et entre dans le compartiment liquide de dialyse par l'entrée 9. Ainsi, le liquide de rinçage circule dans les deux compartiments 3 et 4 du dialyseur dans la même direction, de bas en haut, ce qui facilite l'évacuation de l'air contenu dans le dialyseur 2.

Après avoir rincé le compartiment liquide de dialyse 4, le liquide s'écoule dans la canalisation de raccordement 18, ce qui provoque l'augmentation de la pression dans cette canalisation, la vanne 21 étant fermée. Lorsque la pression existant dans la canalisation de raccordement 18, mesurée par le capteur20, dépasse un seuil prédéterminé (par exemple, 80 mm Hg), l'ouverture de la vanne 21 est commandée par l'unité 55. Le liquide de rinçage s'écoule alors dans la canalisation d'évacuation 35 où il est mis en circulation avec le liquide de dialyse en préparation par la pompe 36. Par la suite, la valve 21 reste commandée en fonction de la pression mais son état (ouvert/fermé) ne dépend plus de la valeur absolue de la pression dans la canalisation de raccordement 18 : il devient alors fonction de la différence de pression ΔP entre la pression Pi existant dans la canalisation de raccordement 18 et la pression P0 existant dans la canalisation d'évacuation 34, de façon que le liquide de rinçage s'écoule en permanence dans la canalisation d'évacuation 34 (ce qui élimine le risque que le liquide de dialyse en préparation ne vienne au contact du dialyseur 2).

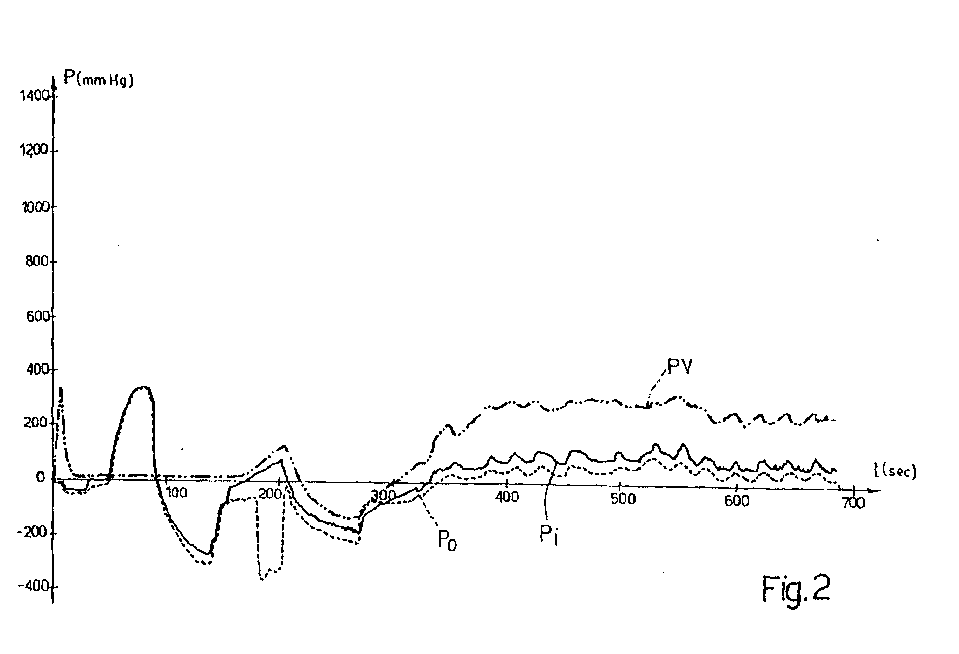

Dans la pratique, lorsque ΔP = Pi - P0 < 30 mm Hg, la vanne 21 est fermée et, lorsque ΔP > 50 mm Hg, la vanne 21 est ouverte. La courbe des pressions Pi et P0 dans les tuyauteries 18 et 34, résultant de ce contrôle est visible sur la figure 2 où (à partir de t = 350 s) on peut voir une progression ondulatoire des deux pressions, due à l'ouverture et à la fermeture continue de la valve 21. Sur la figure 2 il est donné également l'allure de la courbe de pression PV dans la ligne veineuse 15.

La commande de la vanne 21 de la façon qui vient d'être exposée provoque une série d'ondes de pression à l'intérieur du dialyseur 2, dans la ligne veineuse 15 et dans la canalisation de raccordement 18, ce qui facilite le décollement des bulles d'air accrochées à la membrane du dialyseur 2 et aux parois des canalisations et leur évacuation.

A la fin de la procédure de rinçage, le récipient 14 est désolidarisé de la ligne artérielle 11, qui peut être alors reliée au circuit vasculaire d'un patient au moyen d'une canule. La portion de canalisation 17 est déconnectée du dialyseur 2 et est désolidarisée de la ligne veineuse 15, qui peut être alors reliée au circuit vasculaire du patient au moyen d'une canule. La première vanne 35 est fermée, le raccord 48 servant à relier les deux tronçons de la canalisation d'évacuation 34 est ouvert et le second tronçon de la canalisation d'évacuation 34 est relié à l'orifice du compartiment liquide de dialyse auquel était préalablement connectée la portion de canalisation 17. De la sorte, lors de la séance de dialyse, le liquide de dialyse sortant de la deuxième chambre de l'ultrafiltre 22 s'écoule dans la canalisation de raccordement 18, dans le compartiment 4 (dans une direction opposée à celle de la flèche de la figure 1) et, dans la conduite d'évacuation 34.

La présente invention n'est pas limitée aux modes de réalisation décrits et représentés, et elle est susceptible de variantes. En particulier, le procédé décrit peut aussi bien être mis en oeuvre quand deux circuits de rinçage indépendants sont utilisés pour le rinçage des deux compartiments du dialyseur.

## Revendications

1. Dispositif de rinçage d'un appareil de traitement du sang (22) ayant un premier et un second compartiments (3, 4) séparés par une membrane semi-perméable (5) comprenant un circuit de rinçage comprenant :
- des moyens d'alimentation (14) d'au moins un compartiment (3 ; 4) en liquide de rinçage;
- des moyens d'évacuation (18, 34) du liquide de rinçage usé;
- des moyens (21, 55) pour créer des ondes de pression à intervalles de temps dans le circuit de rinçage de façon à décoller des bulles d'air adhérant à une paroi interne du dispositif de traitement de sang (2) et à évacuer ces bulles d'air dans le liquide de rinçage;
- des moyens de pompage (12) pour faire circuler le liquide de rinçage dans le circuit de rinçage ;
- les moyens pour créer des ondes de pression comprennent des moyens d'obturation (21) pour interdire sélectivement la circulation de liquide dans le circuit de rinçage, ces moyens d'obturation (21) étant situés en aval, respectivement en amont, de l'appareil à membrane (2) tandis que les moyens de pompage (12) sont situés en amont, respectivement en aval, de l'appareil à membrane (2);
**caractérisé en ce que** :
- les moyens d'évacuation comprennent une canalisation de raccordement (18) d'une sortie d'un compartiment (3; 4) de l'appareil à membrane (2) à une canalisation d'évacuation (34) d'un générateur (1) de liquide de traitement,
- des moyens de commande (55) sont prévus pour commander l'ouverture et la fermeture des moyens d'obturation (21) de façon à créer des ondes de presssion dans le circuit de rinçage ;
- il comporte des premiers moyens (20) de mesure de pression pour mesurer la pression Pi à l'intérieur d'une portion du circuit de rinçage située entre les moyens de pompage (12) et les moyens d'obturation (21) et des seconds moyens (37) de mesure de pression pour mesurer la pression P0 à l'intérieur de la canalisation d'évacuation (34),
- les moyens de commande (55) sont prévus pour commander l'ouverture et la fermeture des moyens d'obturation (21) en fonction des pressions mesurées ;
- les moyens de commande (55) sont prévus pour commander l'ouverture des moyens d'obturation (21) lorsque la différence de pression Pi - P0 = ΔP est supérieure à une première valeur de seuil prédéterminée et la fermeture des moyens d'obturation (21) est commandée lorsque la différence de pression ΔP est inférieure à une seconde valeur de seuil prédéterminée.

2. Procédé de rinçage d'un appareil de traitement du sang (2) ayant un premier et un second compartiments (3, 4) séparés par une membrane semi-perméable (5), consistant à :
- agencer un circuit de rinçage en reliant une entrée du premier compartiment (3) à une source de liquide de rinçage (14) et une sortie de ce compartiment (3) à des moyens d'évacuation (18, 34) ;
- faire circuler du liquide de rinçage dans le circuit de rinçage par des moyens de pompage (12) disposée sur le circuit de rinçage du côté de l'entrée, respectivement de la sortie, de ce compartiment (3) ;
- créer des ondes de pression à intervalles de temps dans le circuit de rinçage en ouvrant et en fermant alternativement des moyens d'obturation (21) disposés sur le circuit de rinçage du côté de la sortie, respectivement de l'entrée, de ce compartiment (3) de façon à décoller des bulles d'air adhérant à une paroi interne de l'appareil de traitement de sang (2) et à evacuer ces bulles d'air dans le liquide de rinçage, **caractérisé en ce que** :
- le circuit de rinçage est agencé en reliant la sortie du premier compartiment sang (3) à une canalisation d'évacuation (34) d'un générateur (1) de liquide de traitement ; et **en ce que**
- l'ouverture et la fermeture des moyens d'obturation (21) sont commandées en fonction d'une pression Pi mesurée dans une portion circuit de rinçage comprise entre les moyens de pompage (12) et les moyens d'obturation (21).

3. Procédé de rinçage selon la revendication 2, **caractérisé en ce que**:
- la pression P0 est mesurée dans la canalisation d'évacuation (34) ;
- la différence ΔP = Pi - P0 des pressions dans le circuit de rinçage et dans le canalisation d'évacuation (34) est calculée ; et
- l'ouverture des moyens d'obturation (21) est commandée lorsque la différence de pression ΔP est supérieure à une première valeur de seuil prédéterminée et la fermeture des moyens d'obturation est commandée lorsque la différence de pression ΔP est inférieure à une seconde valeur de seuil prédéterminée.

## Patentansprüche

1. Vorrichtung zum Spülen einer Blutbehandlungsvorrichtung (22) mit einem ersten und einem zweiten Kammer (3, 4), die durch eine semipermeable Membran (5) getrennt sind, mit einem Spülkreislauf der folgendes umfasst:
- Mittel zum Versorgung (14) zumindest einer Kammer (3; 4) mit Spülflüssigkeit;
- Mittel zur Abführung (18, 34) der gebrauchten Spülflüssigkeit;
- Mittel (21, 55) zur Erzeugung von Druckwellen bei Zeitintervallen in dem Spülkreislaufs, um an einer Innenwand der Blutbehandlungsvorrichtung (2) anhaftende Luftblasen abzutrennen und diese Luftblasen in die Spülflüssigkeit zu Abführen;
- Pumpmittel (12) zum Fliessen der Spülflüssigkeit im Spülkreislauf,
- die Mittel zur Erzeugung von Druckwellen umfassen Verschlussmittel (21) zum selektiven Unterbinden des Fliessens von Flüssigkeit im Spülkreislauf, wobei diese Verschlussmittel (21) sich stromabwärts bzw. stromaufwärts von der Membranvorrichtung (2) befinden, während die Pumpmittel (12) stromaufwärts bzw. stromabwärts von der Membranvorrichtung (2) liegen;
**dadurch gekennzeichnet, daß**:
- die Abführungsmittel eine Leitung zur Verbindung (18) eines Ausgangs einer Kammer (3; 4) der Membtranvorrichtung (2) mit einer Abflussleitung (34) eines Behandlungsflüussigkeitsgenerators (1) umfassen,
- es Steuermittel (55) zur Steuerung der Öffnung und des Schließens der Verschlussmittel (21) vorgesehen sind, um Druckwellen innerhalb des Spülkreislaufs zu erzeugen;
- sie erste Druckmessmittel (20) zur Messung des Drucks Pi innerhalb eines zwischen den Pumpmitteln (12) und den Verschlussmitteln (21) liegenden Teils des Spülkreislaufs, und zweite Druckmessmittel (37) zur Messung des Drucks P0 innerhalb der Abflussleitung (34) umfasst,
- die Steuermittel (55) zur Steuerung der Öffnung und des Schließens der Verschlussmittel (21) abhängig von den gemessenen Drücken vorgesehen sind;
- die Steuermittel (55) vorgesehen sind zur Steuerung der Öffnung der Verschlussmittel (21), wenn die Druckdifferenz Pi-P0 = ΔP größer als ein erster vorgegebener Grenzwert ist, und des Schließens der Verschlussmittel (21), wenn der Druckdifferenz ΔP kleiner als ein zweiter vorgegebener Grenzwert ist.

2. Verfahren zum Spülen einer Blutbehandlungsvorrichtung (2) mit einem ersten und einem zweiten Kammer (3, 4), die durch eine semipermeable Membran (5) getrennt sind, umfassend die folgenden Schritte:
- Anordnung eines Spülkreislaufs durch Verbinden eines Eingangs der ersten Kammers (3) mit einer Spülffüssigkeitsquelle (14) und eines Ausgangs dieser Kammer (3) mit Ableitungsmitteln (18, 34);
- Zirkulieren von Spülflüssigkeit innerhalb des Spülkreislaufs durch Pumpmittel (12), die im Spülkreislauf an der Eingangs- bzw. Ausgangsseite dieses Kammers (3) angeordnet sind;
- Erzeugung von Druckwellen bei Zeitintervallen innerhalb des Spülkreislaufs durch abwechselndes Öffnen und Schließen der Verschlussmittel (21), die im Spülkreislauf an der Ausgangs- bzw. Eingangsseite dieser Kammer (3) angeordnet sind, um an einer Innenwand der Blutbehandlungsvorrichtung (2) anhaftende Luftblasen abzulösen und diese Luftblasen in die Spülflüssigkeit abzuführen, **dadurch gekennzeichnet, daß**:
- der Spülkreislauf durch Anschließen des Ausgangs der ersten Kammer (3) mit einer Abflussleitung (34) eines Behandlungsflüssigkeitsgenerators (1) ausgelegt ist; und daß
- das Öffnen und Schließen der Verschlussmittel (21) abhängig von einem Druck, der in einem Teil des Spülkreislaufs zwischen den Pumpmitteln (12) und den Verschlussmitteln (21) gemessen wird, gesteuert werden.

3. Spüfverfahren nach Anspruch 2, **dadurch gekennzeichnet, daß**:
- der Druck P0 in der Abflussleitung (34) gemessen wird:
- die Differenz ΔP = Pi-P0 zwischen den Drücken im Spülkreislauf und in der Abflussleitung (34) berechnet wird; und
- die Öffnung der Verschlussmittel (21) gesteuert wird, wenn die Druckdifferenz ΔP größer als ein erster vorgegebener Grenzwert ist, und das Schließen der Verschlussmittel gesteuert wird, wenn die Druckdifferenz ΔP kleiner als ein zweiter vorgegebener Grenzwert ist.

## Claims

1. A device for flushing a blood treatment apparatus (22) having a first and a second compartment (3, 4) separated by a semipermeable membrane (5), this device comprising a flushing circuit which has:
- means (14) for supplying flushing liquid to at least one compartment (3; 4);
- means (18, 34) for discharging the flushing liquid which has been used;
- means (21, 55) for creating pressure waves at time intervals in the flushing circuit, in such a way as to dislodge air bubbles adhering to an inner wall of the blood treatment device (2) and to discharge these air bubbles in the flushing liquid;
- pumping means (12) for circulating the flushing liquid through the flushing circuit;
- the means for creating pressure waves comprises occlusion means (21) for selectively prohibiting the circulation of liquid in the flushing circuit, this occlusion means (21) being situated downstream or upstream of the membrane apparatus (2), while the pumping means (12) is situated upstream or downstream of the membrane apparatus (2);
**characterized in that**:
- the discharge means comprises a pipe (18) connecting an outlet of one compartment (3; 4) of the membrane apparatus (2) to a discharge pipe (34) of a treatment liquid generator (1),
- control means (55) is designed for controlling the opening and closing of the occlusion means (21), in such a way as to create pressure waves in the flushing circuit;
- it includes first pressure measuring means (20) for measuring pressure Pi inside a section of the flushing circuit situated between the pumping means (12) and the occlusion means (21), and second pressure measuring means (37) for measuring the pressure Po inside the discharge pipe (34),
- the control means (55) is designed to control the opening and dosing of the occlusion means (21) as a function of the measured pressures;
- the control means (55) is designed to control the opening of the occlusion means (21) when the pressure difference Pi - Po = ΔP is greater than a first predetermined threshold value, and the closing of the occlusion means (21) is controlled when the pressure difference ΔP is less than a second predetermined threshold value.

2. A method for flushing a blood treatment apparatus (2), which has a first and a second compartment (3, 4) separated by a semipermeable membrane (5), consisting in:
- setting up a flushing circuit by connecting an inlet of the first compartment (3) to a flushing liquid source (14) and an outlet of this compartment (3) to discharge means (18, 34);
- circulating flushing liquid through the flushing circuit by pumping means (12) arranged on the flushing circuit on the inlet side, or outlet side respectively, of said compartment (3);
- creating pressure waves at time intervals in the flushing circuit by alternately opening and closing occlusion means (21) arranged on the flushing circuit on the outlet side, or on the inlet side respectively, of said compartment (3), in such a way as to dislodge air bubbles adhering to an inner wall of the blood treatment apparatus (2) and to discharge these air bubbles in the flushing liquid, **characterized in that**:
- the flushing circuit is set up by connecting the outlet of the first blood compartment (3) to a discharge pipe (34) of a treatment liquid generator (1); and **in that**
- the opening and closing of the occlusion means (21) is controlled as a function of a pressure Pi measured in a flushing circuit section located between the pumping means (12) and the occlusion means (21).

3. The flushing method according to claim 2, **characterized in that:**
- the pressure Po is measured in the discharge pipe (34);
- the difference ΔP = Pi - P0 of the pressures in the flushing circuit and in the discharge pipe (34) is calculated; and
- the opening of the occlusion means (21) is controlled when the pressure differ ence ΔP is greater than a first predetermined threshold value, and the closing of the occlusion means is controlled when the pressure difference ΔP is less than a second predetermined threshold value.
